# FASCICULE DE BREVET EUROPEEN

(11) **EP 2 819 677 B1**
(45) Date de publication et mention de la délivrance du brevet: **21.02.2018**
(21) Numéro de dépôt: 13711080.5
(22) Date de dépôt: 28.02.2013
(51) Int. Cl.: A61K 31/575, A61K 31/57, A61K 31/56, A61K 31/5415, A61K 45/06, A61K 9/00, A61P 15/18

(54) **COMBINAISON DE MODULATEURS SELECTIFS DU RECEPTEUR A LA PROGESTERONE ET D'ANTI-INFLAMMATOIRES NON STEROIDIENS**
KOMBINATION AUS SELEKTIVEN PROGESTERONREZEPTORMODULATOREN UND NICHTSTEROIDEN ENTZÜNDUNGSHEMMENDEN ARZNEIMITTELN
COMBINATION OF SELECTIVE PROGESTERONE-RECEPTOR MODULATORS AND NONSTEROIDAL ANTI-INFLAMMATORY DRUGS

(30) Priorité: 28.02.2012 FR 1251771
(43) Date de publication de la demande: 07.01.2015
(73) Titulaire: Laboratoire HRA Pharma, 75003 Paris (FR)
(72) Inventeur: RESCHE-RIGON, Michèle, F-75011 Paris (FR); LEVY, Delphine, F-93170 Bagnolet (FR); GAINER, Erin, F-75019 Paris (FR)
(74) Mandataire: Verhaeghe Bect, Elodie
(86) Numéro de dépôt international: PCT/FR2013/050424
(87) Numéro de publication internationale: WO 2013/128134

(56) Documents cités:
- EP-A1- 2 263 665
- WO-A1-2006/121969
- WO-A1-2010/149273
- US-A1- 2004 058 975
- FUREDI ET AL: "New emergency contraceptive method ellaOne<(>R) @? is it worth the price?", REPRODUCTIVE HEALTH MATTERS, ELSEVIER, vol. 17, no. 34, 1 novembre 2009 (2009-11-01), pages 187-188, XP026863598, ISSN: 0968-8080 [extrait le 2009-11-01]
- GLASIER A F ET AL: "Ulipristal acetate versus levonorgestrel for emergency contraception: a randomised non-inferiority trial and meta-analysis", THE LANCET, LANCET LIMITED. LONDON, GB, vol. 375, no. 9714, 13 février 2010 (2010-02-13), pages 555-562, XP026904226, ISSN: 0140-6736, DOI: 10.1016/S0140-6736(10)60101-8 [extrait le 2010-01-29]
- JEFFREY T JENSEN: "The future of contraception: innovations in contraceptive agents: tomorrow's hormonal contraceptive agents and their clinical implications", AMERICAN JOURNAL OF OBSTETRICS & GYNECOLOGY, MOSBY, ST LOUIS, MO, US, vol. 205, no. 4, 10 juin 2011 (2011-06-10) , pages S21-S25, XP028300911, ISSN: 0002-9378, DOI: 10.1016/J.AJOG.2011.06.055 [extrait le 2011-06-20]
- ESPEY L L: "FEMALE CONTRACEPTION: NOVEL METHODS AND DEVELOPMENTS", CURRENT OPINION IN THERAPEUTIC PATENTS, XX, XX, vol. 4, no. 6, 1 janvier 1994 (1994-01-01) , pages 629-639, XP009011827, ISSN: 0962-2594
- "Selective progesterone receptor modulator", Wikipedia , 31 mai 2012 (2012-05-31), XP002694887, Extrait de l'Internet: URL:http://en.wikipedia.org/wiki/Selective _progesterone_receptor_modulator [extrait le 2012-09-25]

## Description

La présente invention concerne une méthode de contraception.

### Arrière plan technologique :

L'ulipristal acétate est un modulateur sélectif du récepteur à la progestérone qui inhibe ou retarde l'ovulation. (Stratton et al., Human Reproduction, 2000, 15(5) : 1092-1099) L'ulipristal acétate est le principe actif de la pilule EllaOne® commercialisée pour la contraception d'urgence (Furedi et al., Reproductive Health Matters 2009, 17(34), pages 187-188; Glasier et al., The Lancet, 2010, 375 (9714), pages 555-562). La contraception d'urgence permet d'éviter la survenue d'une grossesse non désirée après un rapport sexuel non ou mal protégé, c'est-à-dire en l'absence d'une autre méthode contraceptive ou en cas de mauvais emploi ou d'échec de la méthode contraceptive utilisée. Des études cliniques ont montré que l'ulipristal acétate, administré en une dose unique de 30mg, est sans danger et efficace pour éviter une grossesse non désirée après un rapport sexuel non ou mal protégé (contraception d'urgence) quand il est administré dans les 72 ou 120 heures après le rapport sexuel. (Creinin et al., Obstetrics and Gynecology, 2006, 108(5) : 1089-1097 ; Glasier et al, Lancet. 2010, 375(9714):555-62 ; Fine et al, Obstet Gynecol. 2010, 115:257-63).

L'ulipristal acetate est également développé pour une contraception à la demande (demande internationale de brevet WO 2010/119029). La contraception dit « à la demande » permet d'éviter la survenue d'une grossesse non désirée par administration d'ulipristal acétate seulement quand cela est nécessaire, c'est-à-dire quand un rapport sexuel est attendu ou quand celui-ci vient de se produire, l'administration d'ulipristal acétate pouvant être répétée au cours du cycle menstruel.

Certains composés anti-inflammatoires non stéroidiens (NSAID) ont aussi été proposés comme contraceptifs. En effet des études ont montré que les anti-inflammatoires non stéroïdiens jouent un rôle dans la réduction ou la prévention de l'ovulation chez différentes espèces animales (rat, lapin, mouton, singe). (Zanagnolo et al., Fertility and Sterility, 1996, 65 : 1036-1043 ; Murdoch, Prostaglandins, 1996, 52 : 497-506). De plus, il a été montré que les anti-inflammatoires non stéroïdiens pouvaient bloquer l'ovulation chez la femme. (Killick et Elstein, Fertility and Sterility, 1987, 47 : 773-777) Plus spécifiquement, d'autres études ont montré que des inhibiteurs spécifiques des cyclo-oxygénases de type 2 retardent la rupture folliculaire et donc l'ovulation chez la femme. (Pall et al., Human Reproduction, 2001, 16 : 1323-1328 ; Bata et al., Journal of Clinical Pharmacology, 2006, 46(8) : 925-932 ; Jesam et al., Human Reproduction, 2010, 25(2) : 368-373). Des études chez les rongeurs et le bovins ont montré que la production de prostaglandines au moment de l'ovulation, sous l'impulsion des COX-2 exprimées dans les cellules de la granulosa, conduit à une augmentation de la perméabilité vasculaire, et de l'expression et l'activation d'enzymes protéolytiques nécessaires à l'expulsion de l'oocyte (Richards JS, 1994, Endocr Rev, 15 : 725-751 ; Richards JS, et al. 1995, Recent Prog Horm Res, 50 : 223-254). Des compositions pharmaceutiques pour la contraception d'urgence comprenant des inhibiteurs de cyclo-oxygénase et le lévonorgestrel sont décrits dans WO 2010/149273.

### Résumé de l'invention :

Les inventeurs proposent maintenant de combiner au moins un SPRM choisi parmi l'ulipristal acétate ou un de ses métabolites, avec au moins un anti-inflammatoire non stéroïdien. Cette combinaison offre une meilleure efficacité de l'inhibition de l'ovulation.

Un objet de l'invention est donc une composition pharmaceutique comprenant au moins un SPRM choisi parmi l'ulipristal acétate ou un de ses métabolites, tels que définis à la revendication 1 et au moins un composé anti-inflammatoire non stéroïdien choisi parmi un inhibiteur de cyclo-oxygénase de type 2 (COX-2). Un autre objet de l'invention est une méthode de contraception chez la femme, comprenant l'administration d'une combinaison d'au moins un SPRM choisi parmi l'ulipristal acétate ou un de ses métabolites tels que définis à la revendication 1, avec au moins un composé anti-inflammatoire non stéroïdien inhibiteur de cyclo- oxygénase de type 2, de préférence un oxicam, de préférence encore le piroxicam.

Selon un premier mode de réalisation, le SPRM choisi parmi l'ulipristal acétate ou un de ses métabolites tels que définis à la revendication 1, et le composé anti-inflammatoire non stéroïdien sont destinés à une administration séparée, simultanée ou séquentielle.

Selon un autre mode de réalisation, le SPRM choisi parmi l'ulipristal acétate, ou un de ses métabolites tels que définis à la revendication 1, et le composé anti-inflammatoire non stéroïdien sont combinés au sein de la même composition pharmaceutique.

### Figures

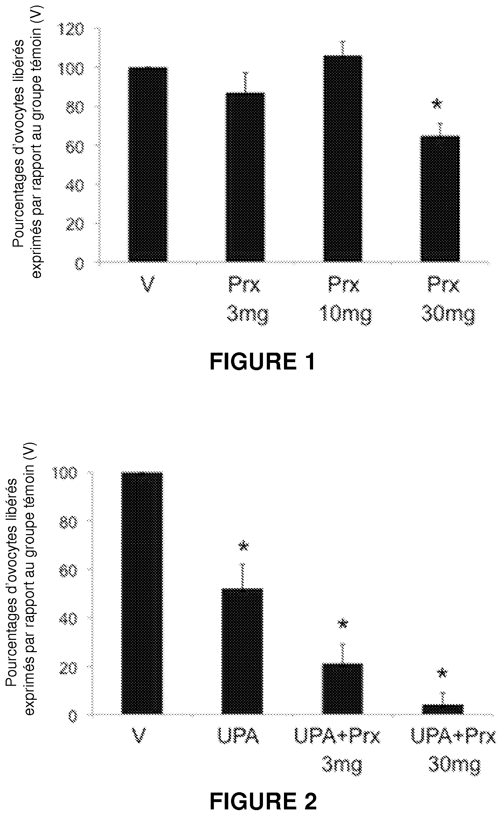

La Figure 1 montre l'effet de l'administration de piroxicam chez des souris ayant subi un traitement d'hyperstimulation ovarienne (superovulation). Les souris sont traitées soit par un véhicule contrôle (V), soit par du piroxicam administré à 3 mg/kg (Prx 3mg), 10 mg/kg (Prx 10 mg), ou 30 mg/kg (Prx 30 mg), 8 heures après l'injection d'hormone chorionique gonadotrope (hCG). Les ovocytes libérés sont comptés 18 heures après l'injection d'hCG. Le diagramme représente, pour chaque groupe, le nombre d'ovocytes libérés exprimés en pourcentages par rapport au groupe témoin (V). Les pourcentages sont représentés en tant que pourcentages moyens ± SEM (écart-type sur la moyenne). La mention* indique que la différence observée par rapport au groupe témoin est statistiquement significative (p < 0,05).
La Figure 2 montre l'effet de l'administration d'ulipristal acétate, seul ou en combinaison avec le piroxicam, chez des souris ayant subi un traitement d'hyperstimulation ovarienne. Le protocole expérimental est identique à celui présenté plus haut. Les groupes expérimentaux sont les suivants : V : groupe témoin ayant reçu le véhicule de contrôle ; UPA : groupe ayant reçu 40 mg/kg d'ulipristal acétate ; UPA + Prx 3 mg : groupe ayant reçu 40 mg/kg d'ulipristal acétate + 3 mg/kg de piroxicam ; UPA + Prx 30 mg : groupe ayant reçu 40 mg/kg d'ulipristal acétate + 30 mg/kg de piroxicam. Le diagramme représente, pour chaque groupe, le nombre moyen d'ovocytes libérés exprimés en pourcentages (± SEM) par rapport au groupe témoin (V). La mention * indique que la différence observée par rapport au groupe témoin est statistiquement significative (p < 0,05).

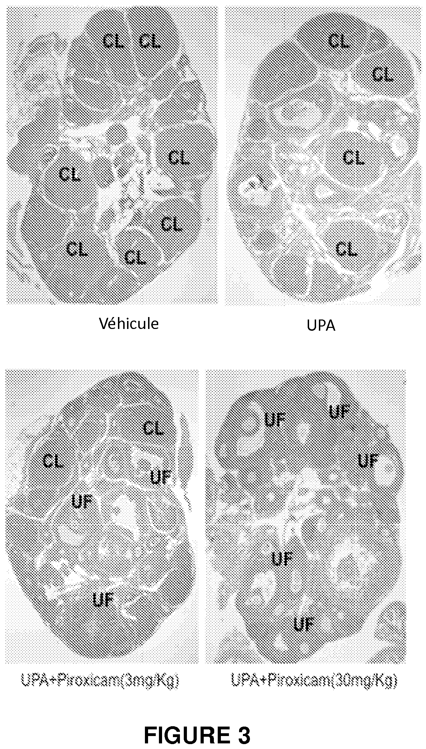

La Figure 3 montre les coupes histologiques ovariennes, après coloration à l'hématoxyline et à l'éosine, provenant d'une souris appartenant au groupe témoin (V), une souris ayant reçu 40 mg/kg d'UPA (UPA), une souris ayant reçu 40 mg/kg d'ulipristal acétate + 3 mg/kg de piroxicam (UPA + Piroxicam 3 mg/kg), et une souris ayant reçu 40 mg/kg d'ulipristal acétate + 30 mg/kg de piroxicam (UPA + Piroxicam 30 mg/kg). CL : corps jaune (*corpus luteum*), UF : follicule non rompu.

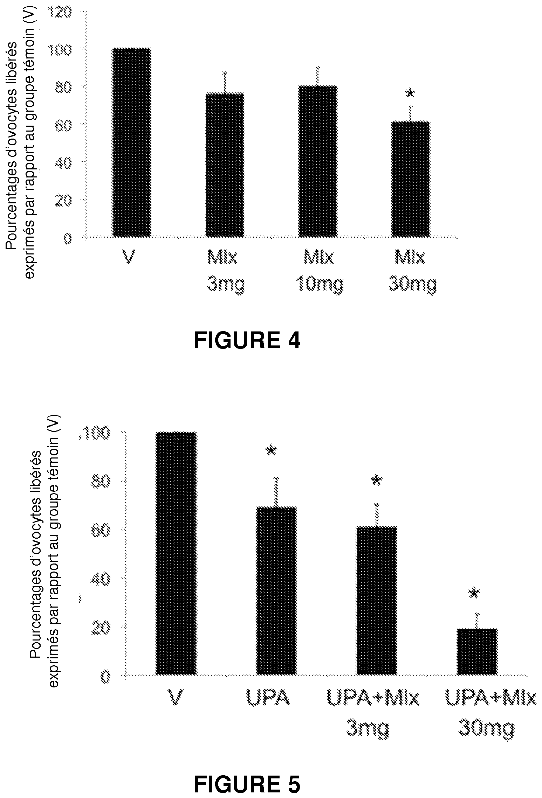

La Figure 4 montre l'effet de l'administration de meloxicam chez des souris ayant subi un traitement d'hyperstimulation ovarienne. Les groupes expérimentaux sont les suivants : V : groupe témoin ; Mlx 3mg : groupe ayant reçu 3 mg/kg de meloxicam ; Mlx 10 mg : groupe ayant reçu 10 mg/kg de meloxicam ; Mlx 30 mg : groupe ayant reçu 30 mg/kg de meloxicam. Le diagramme représente, pour chaque groupe, le nombre moyen d'ovocytes libérés exprimés en pourcentages (± SEM) par rapport au groupe témoin (V). La mention * indique que la différence observée par rapport au groupe témoin est statistiquement significative (p < 0,05).
La Figure 5 montre l'effet de l'administration d'ulipristal acétate, seul, ou en combinaison avec le meloxicam, chez des souris ayant subi un traitement d'hyperstimulation ovarienne. Les groupes expérimentaux sont les suivants : V : groupe témoin ; UPA : groupe ayant reçu 40 mg/kg d'ulipristal acétate ; UPA + Mlx 3 mg : groupe ayant reçu 40 mg/kg d'ulipristal acétate + 3 mg/kg de meloxicam ; UPA + Mlx 30 mg : groupe ayant reçu 40 mg/kg d'ulipristal acétate + 30 mg/kg de meloxicam. Le diagramme représente, pour chaque groupe, le nombre moyen d'ovocytes libérés exprimés en pourcentages (± SEM) par rapport au groupe témoin (V). La mention * indique que la différence observée par rapport au groupe témoin est statistiquement significative (p < 0,05).

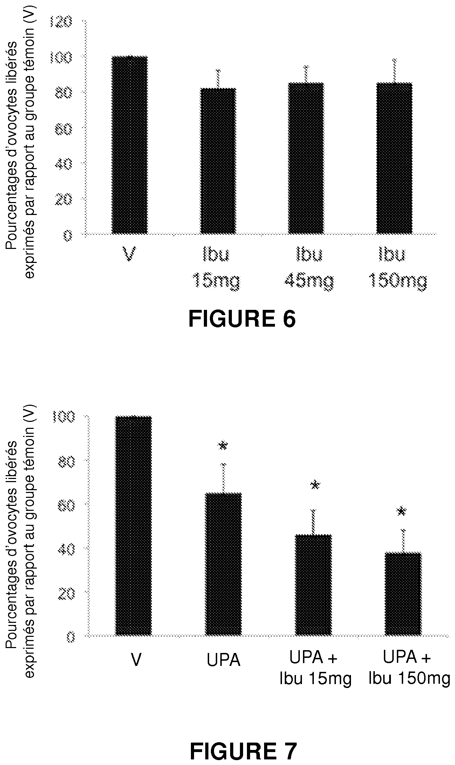

La Figure 6 montre l'effet de l'administration d'ibuprofène chez des souris ayant subi un traitement d'hyperstimulation ovarienne. Les groupes expérimentaux sont les suivants : V : groupe témoin ; Ibu 15 mg : groupe ayant reçu 15 mg/kg d'ibuprofène ; Ibu 45 mg : groupe ayant reçu 45 mg/kg d'ibuprofène ; Ibu 150 mg : groupe ayant reçu 150 mg/kg d'ibuprofène. Le diagramme représente, pour chaque groupe, le nombre moyen d'ovocytes libérés exprimés en pourcentages (± SEM) par rapport au groupe témoin (V). L'étude statistique a montré que les résultats obtenus pour les groupes traités avec l'ibuprofène ne sont pas significativement différents des résultats du groupe témoin.
La Figure 7 montre l'effet de l'administration d'ulipristal acétate, seul ou en combinaison avec l'ibuprofène, chez des souris ayant subi un traitement d'hyperstimulation ovarienne. Les groupes expérimentaux sont les suivants : V : groupe témoin ; UPA : groupe ayant reçu 40 mg/kg d'ulipristal acétate ; UPA + Ibu 15 mg : groupe ayant reçu 40 mg/kg d'ulipristal acétate + 15 mg/kg d'ibuprofène ; UPA + Ibu 150 mg : groupe ayant reçu 40 mg/kg d'ulipristal acétate + 150 mg/kg de ibuprofène. Le diagramme représente, pour chaque groupe, le nombre moyen d'ovocytes libérés exprimés en pourcentages (± SEM) par rapport au groupe témoin (V). La mention * signifie que la différence observée par rapport au groupe témoin est statistiquement significative (p < 0,05).

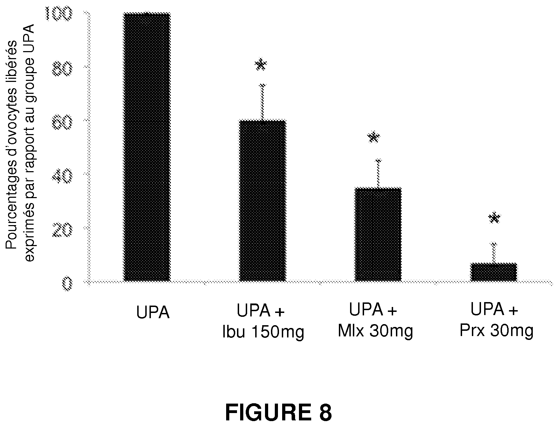

La Figure 8 montre l'effet de l'administration conjointe d'UPA et d'un anti-inflammatoire non stéroïdien (AINS) par rapport à l'administration seule d'UPA chez des souris ayant subi un traitement d'hyperstimulation ovarienne. UPA : groupe ayant reçu 40 mg/kg d'ulipristal acétate ; UPA + Ibu 150 mg : groupe ayant reçu 40 mg/kg d'ulipristal acétate + 150 mg/kg de ibuprofène ; UPA + Mlx 30 mg : groupe ayant reçu 40 mg/kg d'ulipristal acétate + 30 mg/kg de meloxicam ; et UPA + Prx 30 mg : groupe ayant reçu 40 mg/kg d'ulipristal acétate + 30 mg/kg de peroxicam. Le diagramme représente, pour chaque groupe, le nombre moyen d'ovocytes libérés exprimés en pourcentages (± SEM) par rapport au groupe ayant reçu 40 mg/kg d'UPA. L'analyse statistique a montré que les combinaisons UPA-AINS sont significativement plus efficaces pour inhiber la rupture folliculaire que l'administration seule d'UPA (p < 0,05).

### Description détaillée de l'invention :

L'efficacité de l'ulipristal acétate dans la contraception d'urgence repose sur sa capacité à inhiber ou retarder la rupture folliculaire. Brache et al. (Hum Reprod, 2011, 9 : 2256-2263) ont montré qu'une seule administration d'EllaOne® (ulipristal acétate 30mg) survenant après que la production de LH (hormone lutéinisante) ait commencé à augmenter mais avant le pic de LH, conduisait à une inhibition de la rupture folliculaire pendant les 5 jours suivant l'administration dans 78,6% des cas. Cette efficacité chute à 8,3% si l'administration de l'ulipristal acétate survient après le pic de LH. Comme cela est montré en détails dans les exemples, l'administration combinée d'un agent anti-inflammatoire non stéroïdien, en particulier un inhibiteur de cyclo-oxygénases et de l'ulipristal acétate (UPA) permet d'augmenter, et même de potentialiser, la capacité de l'UPA à inhiber la rupture folliculaire.

Sur cette base, les inventeurs proposent de combiner un SPRM avec un anti-inflammatoire non stéroïdien, et de fournir ainsi une nouvelle méthode de contraception, que ce soit une contraception d'urgence ou une contraception régulière, présentant une meilleure efficacité de l'inhibition de l'ovulation.

### Modulateurs du récepteur à la progestérone :

La demande décrit des modulateurs du récepteur à la progestérone, de préférence un modulateur sélectif du récepteur à la progestérone (SPRM). On entend par « modulateur sélectif du récepteur de la progestérone » un ligand du récepteur à la progestérone qui exerce une activité agoniste, une activité antagoniste ou une activité mixte agoniste/antagoniste de manière tissu-spécifique, de préférence une activité agoniste ou agoniste/antagoniste mixte. Au regard de ses connaissances générales, l'homme du métier pourra déterminer par des expériences de routine si un composé est un SPRM, en particulier en se référant aux articles de Smith et O'Malley, Endocrine Review, 25(1) :45-71, et de Chabbert-Buffet et al., Human Reproduction Update,2005, 11, 293-307. Un composé SPRM peut être un composé non-stéroïdien ou un dérivé stéroïdien. Des exemples de modulateurs non-stéroïdiens sélectifs du récepteur à la progestérone sont fournis dans les publications suivantes : Dong et al., Steroids, 2004, 69 :201-207, Zhi et al., J Med Chem, 2003, 46 :4104-4112 et Zhi et al., Curr Top Med Chem, 2008, 8 :766-780. Des exemples de composés SPRM stéroïdiens peuvent être choisi parmi les dérivés stéroïdiens substitués en position 11β par un groupe aryle. Les groupes aryles adaptés comprennent, sans y être limités, le 4-(diméthylamino)phényle, le 4-acétylphényle, et la benzaldoxime.

Pour mémoire, les atomes de carbone du noyau stéroïde sont numérotés de la manière suivante :

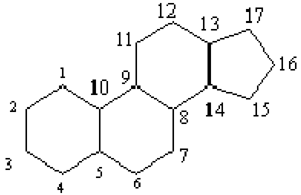

Des exemples de SPRMs stéroidiens sont fournis dans les publications suivantes : Rao et al., Steroids, 1998, 63:523-530 et Chabbert-Buffet et al., Human Reproduction Update,2005, 11, 293-307. En particulier, Chabbert-Buffet et al. cite la mifépristone, l'onapristone, l'asoprisnil, l'acétate d'ulipristal, Org 33628 et Org 31710 comme étant des SPRM. Un SPRM stéroïdien peut être un composé de de formule (I) suivante : 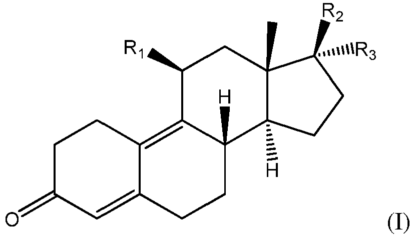 dans laquelle :
- R₁ représente un groupe aryle éventuellement substitué par un ou plusieurs groupes indépendamment en position ortho, para ou méta, de préférence choisis parmi le groupe constitué par H, OH, -CH=N-OH, -C(=O)-R₄, un groupe alcoxy en C1-C5, un groupe alkylamine en C1-C5 ou un groupe dialkylamine en C1-C5,
- R₂ représente -OH, un groupe alcoxy en C1-C5 ou -C(=O)-R₄, et
- R₃ représente -OH, un groupe alcoxy en C1-C5, un alcynyle en C2-C5, un alcényle en C2-C5 ou -O-C(=O)-R₄,
   R₄ étant choisi parmi un groupe alkyle en C1-C3 et un groupe alcoxy en C1-C5. et leurs métabolites et sels pharmaceutiquement acceptables.

De préférence, R₃ représente -OH, un groupe alcoxy en C1-C5, un alcynyle en C2-C5, ou -O-C(=O)-R₄. Un alkyle en C1-C3 englobe les groupes méthyle, éthyle, propyle et isopropyle.

Un groupe alcoxy en C1-C5 englobe les groupes de formule -(CH₂)ₙO(CH₂)₍₄₋ₙ₎CH₃, n étant un entier allant de 0 à 4. Un SPRM peut être un composé de formule (la) suivante, ainsi que ses métabolites et sels pharmaceutiquement acceptables : 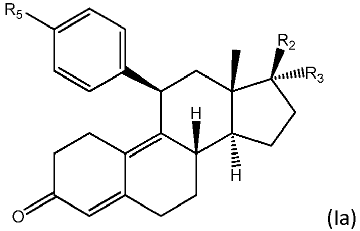 Dans laquelle :
- R₅ représente :
   - -NR₆R₇ dans lequel R₆ et R₇ représente, indépendamment l'un de l'autre, -H ou un alkyle en C1-C3, R₆ et R₇ étant choisis de préférence parmi H et -CH₃ ;
   - -CH=N-OR₈ dans lequel R₈ représente -H ou -C(=O)-X-R₉ avec R₉ étant un alkyle en C₁-C₃ et X représentant O, NH ou S ; ou
   - -C(=O)R₁₀ dans lequel R₁₀ représente un alkyle en C₁-C₃
- R₂ et R₃ étant tels que définis précédemment.

De préférence, R₃ représente -OH, un groupe alcoxy en C1-C5, un alcynyle en C2-C5, ou -O-C(=O)-R₄ avec R₄ étant choisi parmi un groupe alkyle en C1-C3 et un groupe alcoxy en C1-C5

De tels composés comprennent, sans y être limités, la mifépristone, l'ulipristal acétate, l'asoprisnil et la télapristone. En particulier, la mifépristone correspond au composé de formule (la) dans laquelle R5 est -N(CH₃)₂, R₂ est OH et R₃ est -C≡C-CH₃. Y sont compris des composés SPRMs de formule (la) dans laquelle :
- R₂ représente -OH, -OCH₃, -C(=O)CH₃ ou -(C=O)-CH₂-O-CH₃
- R₃ représente -CH₂-O-CH₃, ou -O-C(=O)-CH₃
- R₅ représente -NH₂, -NHCH₃, -N(CH₃)₂ ou -CH=N-OR₈ dans lequel R₈ représente H, -(C=O)-S-C₂H₅ ou -C(=O)-NH-C₂H₅. Y sont également compris les composés de formule (Ia) dans laquelle R₅ est -CH=NOR₈. De tels composés englobent :
   - Asoprisnil (R₈ est H, R₂ est OMe et R₃ est -CH₂OMe),
   - J912 (R₈ est H, R₂ est H et R₃ est -CH₂OMe),
   - J956 (également appelé Asoprisnil ecamate) (R₈ est -C(=O)-NH-C₂H₅, R₂ est-OMe et R₃ est -CH₂OMe), et
   - J1042 (R₈ est -C(=O)S-C₂H₅, R₂ est -OCH₃ et R₃ est -CH₂OMe). Y sont enfin compris les composés de formule (Ia) dans laquelle R₅ est -N(Me)₂. De tels composés englobent l'ulipristal acétate (R₂ est -C(=O)-CH₃ et R₃ est -O-C(=O)CH₃) et la télapristone (Proellex® également appelé CDB-4124) (R₂ est -C(=O)-CH₂-O-CH₃ et R₃ est -O-C(=O)-CH₃). Selon l'invention, le composé SPRM est l'ulipristal acétate (également appelé CDB-2914) ou l'un de ses métabolites tels que définis ci-après. L'ulipristal acétate est le 17α-acétoxy-11β-[4-N, N-diméthylamino-phényl)-19-norprégna- 4, 9-diène-3, 20-dione (nomenclature IUPAC), représenté par la formule I :

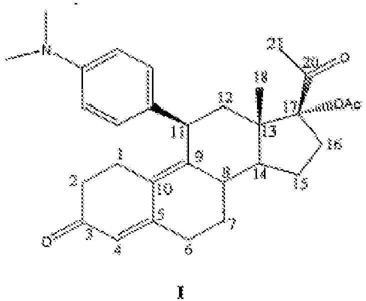

Ce composé et les procédés d'obtention de ce composé sont décrits dans les brevets américains US 4,954,490 ; US 5,073,548 et US 5,929,262 et demandes internationales WO 2004/065405 et WO 2004/078709 notamment.

On peut également utiliser un des métabolites de l'ulipristal acétate, tels que décrits dans Attardi et al., Journal of Steroid Biochemistry and Molecular Biology, 2004, 88 : 277-288. Les métabolites de l'ulipristal acétate englobent, entre autres :
- le composé CDB-3877 (composé de formule (Ia) dans laquelle R₅ est -NHCH₃, R₂ est -C(=O)-CH₃ et R₃ est -O-C(=O)-CH₃) et
- le composé CDB-3963 (composé de formule (Ia) dans laquelle R₅ est -NH₂, R₂ est -C(=O)-CH₃ et R₃ est -O-C(=O)-CH₃.

Les métabolites de l'ulipristal acétate décrits par Attardi et al. sont les suivants :

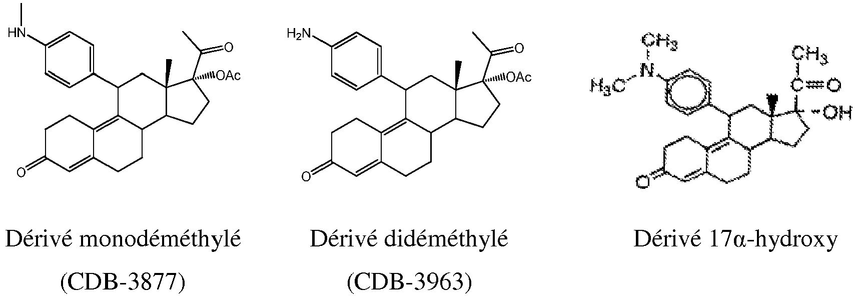

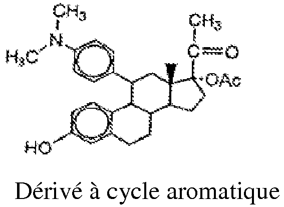

Le métabolite de l'ulipristal acétate est de préférence le composé CDB-3877 ou le composé CDB-3963, de manière encore plus préférée, le composé CDB-3877.

### Les anti-inflammatoires non stéroïdiens :

Le terme « AINS » ou en anglais « NSAID » se réfère à tout composé anti-inflammatoire non stéroïdien. Ces composés sont groupés selon leur capacité à inhiber une cyclooxygénase. Les cyclooxygénases 1 et 2 sont les deux isoformes principales et la plupart des AINS sont des inhibiteurs mixtes des deux isoformes. Cinq catégories d'inhibiteurs mixtes sont généralement distinguées : (1) dérivés d'acide propionique, comme ibuprofène, naproxène, naprosyn, diclofénac, et ketoprofen; (2) dérivés d'acide acétique comme tolmetin et slindac; (3) dérivés d'acide fénamique comme l'acide mefenamique et l'acide meclofenamique; (4) les dérivés d'acide biphenylcarboxylique comme le diflunisal et le flufenisal; et (5) les oxicams, comme meloxicam, piroxicam, sudoxicam, et isoxicam.

Des exemples de composés inhibiteurs sélectifs de COX-2 incluent celecoxib (SC-58635), DUP-697, flosulide (CGP-28238), meloxicam, acide 6-methoxy-2 naphthylacetique (6-MNA), rofecoxib, MK-966, nabumetone, nimesulide, NS-398, SC-5766, SC-58215, T-614.

Dans certains modes de réalisation de l'invention, le composé anti-inflammatoire non-stéroïdien est choisi parmi les oxicams, les dérivés de l'acide propionique, de préférence les dérivés de l'acide 2-arylpropionique, et leurs combinaisons.

Ainsi, le composé anti-inflammatoire non-stéroïdien peut être choisi parmi les oxicams. Les oxicams comprennent notamment le piroxicam, le tenoxicam, le droxicam, le lornoxicam, le meloxicam, le sudoxicam, et les combinaisons de ceux-ci.

Le composé anti-inflammatoire non-stéroïdien peut être également choisi parmi les dérivés de l'acide propionique qui comprennent, entre autre, l'ibuprofène, le naproxène, le naprosyn, le diclofénac, et le kétoprofène.

De préférence, l'agent anti-inflammatoire non-stéroïdien est choisi parmi le meloxicam, le piroxicam et leurs combinaisons. Alternativement, dans l'invention, le composé anti-inflammatoire non stéroïdien est un inhibiteur de cyclo-oxygénase de type 2 (COX-2) choisi dans le groupe consistant en l'indométacine, le naproxène, l'ibuprofène, l'acétominophène, le meloxicam, le piroxicam, l'étodolac et le célécoxib. Des dérivés de ces composés sont également inclus, tels que par exemple le naproxinod, qui est composé du naproxène ainsi qu'une molécule d'oxyde nitrique.

Au sens de l'invention, un inhibiteur de cyclo-oxygénase de type 2 (COX-2) est un inhibiteur sélectif ou non-sélectif de COX-2.

De préférence, on utilise un inhibiteur non sélectif des COX, c'est-à-dire un composé qui inhibe à la fois COX-1 et COX-2 de manière non sélective.

Ainsi on préfère utiliser le piroxicam ou l'ibuprofène comme inhibiteur de COX.

On peut aussi utiliser des inhibiteurs sélectifs de COX-2, tels que notamment le meloxicam.

### Compositions pharmaceutiques :

Le SPRM choisi parmi l'ulipristal acétate ou un de ses métabolites, et l'AINS peuvent être combinés au sein d'une même composition pharmaceutique, ou être utilisés sous forme de compositions pharmaceutiques séparées, administrables de manière simultanée ou séquentielle. En particulier ils peuvent être administrés séparément, à savoir soit concomitamment, soit indépendamment, par exemple, avec un décalage dans le temps.

Conformément à l'invention, le SPRM choisi parmi l'ulipristal acétate ou un de ses métabolites, et l'AINS sont utilisés en combinaison afin de potentialiser les effets de l'ulipristal acetate sur l'inhibition de l'ovulation.

Quelle que soit la voie d'administration et la forme des compositions pharmaceutiques, de préférence les composés sont administrés en des quantités synergiques vis à vis de l'effet anti-ovulation.

Les compositions pharmaceutiques selon l'invention comprennent avantageusement un ou plusieurs excipients ou véhicules, acceptables sur le plan pharmaceutique. On peut citer par exemple des solutions salines, physiologiques, isotoniques, tamponnées, etc., compatibles avec un usage pharmaceutique et connues de l'homme du métier. Les compositions peuvent contenir un ou plusieurs agents ou véhicules choisis parmi les dispersants, solubilisants, stabilisants, conservateurs, etc. Des agents ou véhicules utilisables dans des formulations (liquides et/ou injectables et/ou solides) sont notamment la méthylcellulose, l'hydroxyméthylcellulose, la carboxyméthylcellulose, le polysorbate 80, le mannitol, la gélatine, le lactose, des huiles végétales, l'acacia, etc. Les compositions peuvent être formulées sous forme de suspensions injectables, gels, huiles, comprimés, suppositoires, poudres, gélules, capsules, etc., éventuellement au moyen de formes galéniques ou de dispositifs assurant une libération prolongée et/ou retardée. Pour ce type de formulation, on utilise avantageusement un agent tel que la cellulose, des carbonates ou des amidons.

La combinaison selon l'invention peut être administrée par toute voie d'administration appropriée, par exemple par voie orale, buccale, sublinguale, vaginale, intrautérine, rectale, transdermale ou par voie parentérale, par exemple par injection intraveineuse, intracutanée ou intradermique. De préférence, une administration orale est prévue.

En conséquence le médicament peut se composer sous forme de comprimés, de gélules, de poudre ou de toute forme pour préparation orale solide ou sous toute forme de préparation buvable. La composition pharmaceutique comprend généralement un milieu physiologiquement acceptable, par exemple pour la préparation de comprimés ou de gélules ou pour une préparation buvable telle que les véhicules utilisés de façon tout à fait classique.

Des comprimés particuliers d'ulipristal acetate sont par exemple décrits dans la demande de brevet WO2010/066749.

De préférence, le SPRM, choisi parmi l'ulipristal acétate ou un de ses métabolites, est utilisé à un dosage de 1 mg à 100 mg, 2,5 à 100 mg, de préférence de 5 à 50 mg, de préférence 5 à 30 mg, 15 mg à 35 mg, ou de 10 à 30 mg. L'AINS est lui avantageusement utilisé à un dosage de 5 mg à 1 g par prise, de préférence de 20 à 800 mg par prise, de préférence de 20 mg à 400 mg, de préférence encore de 100 à 200 mg par prise, de préférence en une prise quotidienne. Il est entendu que certains AINS, tels que l'ibuprofène, peuvent être utilisés à des doses de 200 à 600 mg environ. Le piroxicam peut être avantageusement utilisé à un dosage de 20 à 80 mg. Le meloxicam peut être avantageusement utilisé à un dosage de 5 à 60 mg. De préférence, le SPRM, choisi parmi l'ulipristal acétate ou un de ses métabolites, et l'AINS, sont utilisés dans un rapport de 0,01 à 10, de préférence de 5/80 à 30/20, de préférence encore de 0,05/2 environ.

Préférentiellement, la combinaison pharmaceutique selon l'invention comprend environ 30 mg d'ulipristal acétate ou d'un de ses métabolites, et environ 5 à 400 mg d'un ou de plusieurs anti-inflammatoires non stéroïdiens. Une telle combinaison pharmaceutique est destinée préférentiellement à une contraception d'urgence ou à la demande.

En contraception régulière, il est préférable d'utiliser une combinaison pharmaceutique comprenant de 1 mg à 5 mg d'ulipristal acétate.

Les compositions pharmaceutiques, destinées à une administration simultanée, séparée ou séquentielle, peuvent aussi être présentées sous forme de kit, par exemple sous forme de plaquette de pilules contraceptives.

Ainsi un kit contraceptif, peut être élaboré au sein d'un même emballage et comprendre :
- une composition pharmaceutique A comprenant un SPRM choisi parmi l'ulipristal acétate ou un de ses métabolites, dans un milieu physiologiquement acceptable ; et
- une composition pharmaceutique B comprenant un ou plusieurs anti-inflammatoires non stéroïdiens dans un milieu physiologiquement acceptable.

Le kit contraceptif peut comprendre une ou plusieurs unités de dose de la composition pharmaceutique A et une ou plusieurs unités de dose de la composition pharmaceutique B. Le nombre d'unité de doses de chaque composition dépend du schéma posologique choisi et de l'indication contraceptive recherchée, c'est-à-dire une contraception régulière, à la demande ou d'urgence, comme cela est détaillé ci-après.

### Indications :

La combinaison d'un SPRM choisi parmi l'ulipristal acétate ou un de ses métabolites, en association avec un ou plusieurs anti-inflammatoires non stéroïdiens, est utile dans une méthode de contraception chez la femme.

Les termes "méthode de contraception chez la femme" et "contraception chez la femme" font référence à une méthode permettant d'éviter la survenue d'une grossesse non désirée, chez une femme en âge de procréer, lors d'un rapport sexuel. Préférentiellement, l'invention concerne une méthode de contraception d'urgence chez la femme.

Les termes "méthode de contraception d'urgence chez la femme" et "contraception d'urgence chez la femme" font référence à une méthode permettant d'éviter la survenue d'une grossesse non désirée, chez une femme en âge de procréer, après un rapport sexuel non ou mal protégé, c'est-à-dire en l'absence d'une contraception ou en cas d'échec de la méthode contraceptive utilisée.

L'invention concerne aussi une méthode de contraception régulière chez la femme. Si le SPRM est administré par voie orale, la méthode de contraception peut par exemple consister en une administration quotidienne pendant au moins 20 jours dans le cycle, ou tous les jours sans interruption.

L'invention concerne par ailleurs une méthode de contraception à la demande chez la femme.

Les termes "méthode de contraception à la demande chez la femme" et "contraception à la demande chez la femme" font référence à une méthode permettant d'éviter la survenue d'une grossesse non désirée, chez une femme en âge de procréer, par prise du traitement quand cela est nécessaire, c'est-à-dire quand un rapport sexuel est attendu ou quand celui-ci vient de se produire.

La méthode de contraception d'urgence chez la femme selon l'invention, comprend l'administration à un sujet de sexe féminin, en âge de procréer, d'une quantité efficace de SPRM, choisi parmi ou d'un de ses métabolites, en association avec un ou plusieurs anti-inflammatoires non stéroïdiens inhibiteurs de cyclo-oxygénase de type 2, après un rapport sexuel non ou mal protégé. Dans certains cas, comme lorsque le SPRM est de l'uliprital acétate, l'administration peut être réalisée jusqu'à environ 5 jours après un rapport sexuel non ou mal protégé. De préférence la combinaison de l'invention est administrée dans les 120h, de préférence 72h, de préférence 48h, de préférence 24h après le rapport sexuel.

La méthode de contraception à la demande chez la femme selon l'invention, comprend l'administration à un sujet de sexe féminin, en âge de procréer, d'une quantité efficace de SPRM choisi parmi l' ulipristal acétate ou d'un de ses métabolites, en association avec un ou plusieurs anti-inflammatoires non stéroïdiens, entre environ 3 jours, de préférence dans les 24h, de préférence dans les 12h, avant un rapport sexuel non protégé et environ 5 jours, de préférence dans les 120h, de préférence 72h, de préférence 48h, de préférence 24h, après un rapport sexuel non protégé. L'administration peut être répétée au moins une fois par semaine, voire plusieurs fois par mois, ou encore plusieurs fois par semaine.

Une telle méthode de contraception « à la demande » peut remplacer une méthode de contraception hormonale régulière classique.

Dans les méthodes de contraception selon l'invention, les administrations du SPRM et de l'agent anti-inflammatoire non stéroïdien peuvent être simultanées, séquencées ou séparées, c'est-à-dire étalées dans le temps. L'administration du SPRM peut ainsi précéder ou suivre de plusieurs minutes voire de plusieurs heures l'administration du AINS. De préférence, l'administration du SPRM et celle du AINS sont simultanées ou séparées dans le temps d'au plus 12 heures, d'au plus 6 heures, d'au plus 4 heures, d'au plus 3 heures, d'au plus 2 heures et de préférence d'au plus 1 heure.

Dans les méthodes de contraception d'urgence ou à la demande selon l'invention, l'administration de l'agent anti-inflammatoire non stéroïdien peut être renouvelée une ou plusieurs fois, sur un ou plusieurs jours, après son administration en combinaison avec le SPRM. Par exemple, l'administration de l'AINS peut être renouvelée de 1 à 5 fois à raison d'une dose par jour.

A titre d'exemple non-limitatif, la méthode de contraception d'urgence selon l'invention peut comprendre l'administration combinée du SPRM et de l'AINS simultanément ou séparée dans le temps d'au plus 6 heures, de préférence d'au plus 1 heure, et en option l'administration d'une dose journalière d'AINS sur 1 à 5 jours suivant ladite administration combinée du SPRM et de l'AINS.

Dans un mode préféré de réalisation, l'administration de l'AINS n'est pas répétée. La présente description décrit également un modulateur sélectif du récepteur à la progestérone (SPRM) choisi parmi l'ulipristal acétate ou l'un de ses métabolites, pour une utilisation en tant que contraceptif chez la femme en combinaison avec au moins un composé anti-inflammatoire non-stéroïdien.

Un objet supplémentaire de la description est un produit de combinaison comprenant un SPRM choisi parmi l'ulipristal acétate ou l'un de ses métabolites, et au moins un composé anti-inflammatoire non-stéroïdien pour une utilisation séparée, simultanée ou séquentielle en contraception chez la femme.

Enfin, un objet supplémentaire de la description est l'utilisation d'un SPRM choisi parmi l'ulipristal acétate ou l'un de ses métabolites, et d'au moins un composé anti-inflammatoire non stéroïdien pour la fabrication d'un médicament destiné à la contraception chez la femme.

Le médicament peut se présenter sous la forme d'une ou plusieurs unités de doses, chaque unité de dose comprenant à la fois le SPRM et le composé anti-inflammatoire non-stéroidien. A titre alternatif, le médicament comprend au moins une unité de dose comprenant le SPRM et au moins une unité de dose comprenant le composé anti-inflammatoire non stéroidien.

De préférence, ces différents aspects se réfèrent à la contraception d'urgence. Il va de soi que les modes de réalisations particuliers de ces différents aspects sont tels que décrits précédemment pour la composition, le kit et la méthode contraceptive selon l'invention.

Les exemples ci-après ont pour but d'illustrer l'invention sans pour autant en limiter la portée.

### Exemples

L'effet de l'administration combinée de l'ulipristal acétate (UPA) et d'un agent anti-inflammatoire non-stéroidien (AINS) sur la rupture du follicule ovarien induite par LH a été évalué chez un modèle animal d'hyperstimulation ovarienne (modèle de superovulation).

### a) Effet d'inhibition de l'ovulation du piroxicam en combinaison avec l'ulipristal acétate

Une hyperstimulation de l'ovulation a été réalisée chez des souris immatures CD1 âgées de 24 à 28 jours par injection de 5 UI de PMSG (pregnant mare's sérum gonadotropin) suivie, 48 h plus tard, de 5 UI de hCG (hormone chorionique gonadotrope) par voie intra-péritoneale. Les souris ont été réparties en plusieurs groupes de 10 souris chacun. 8 heures après l'injection de hCG, les souris ont reçu par voir intra-péritonéale soit le ou les composés à tester, soit le placébo (véhicule). Les souris ont été euthanasiées 18 heures après l'injection de hCG. L'effet du ou des composés sur la rupture folliculaire et donc l'ovulation a été déterminé par comptage des ovocytes libérés dans les oviductes. Dans la série d'expériences n°2, un examen morphologique et histologique des ovaires a été réalisé de manière à évaluer la présence de follicules non-rompus et/ou de corps jaunes dans les ovaires de chaque groupe d'animaux.

Ci-dessous sont présentées les séries d'expérience réalisées et les doses administrées à chaque groupe de souris
Série 1 :
   - Groupe 1 : placebo
   - Groupe 2 : 3 mg/ kg de piroxicam
   - Groupe 3 : 10 mg/kg de piroxicam
   - Groupe 4 : 30 mg/kg de piroxicam
Série 2 :
   - Groupe 1 : placebo
   - Groupe 2 : 40 mg/kg d'ulipristal acétate
   - Groupe 3 : 40 mg/kg d'ulipristal acétate + 3 mg/kg de piroxicam
   - Groupe 4 : 40 mg/kg d'ulipristal acétate + 30 mg/kg de piroxicam

### Résultats :

La figure 1 montre le nombre d'ovocytes libérés dans les oviductes pour les différents groupes de souris de la première série d'expériences exprimé en pourcentages moyens par rapport au groupe témoin 1. Si l'administration de 3 mg ou 10 mg de piroxicam ne réduit pas de manière significative le nombre de ovocytes libérés dans les oviductes (et donc l'ovulation), l'administration de 30 mg de piroxicam permet de réduire de manière significative le nombre d'ovulation observées chez les souris du groupe 4 par rapport au groupe témoin.

La figure 2 montre les résultats de comptage des ovocytes récoltés dans les oviductes pour les souris des différents groupes de la série d'expériences 2. Le nombre moyen d'ovocytes libérés chez le groupe de souris ayant reçu 40 mg/kg d'UPA 8 heures après l'injection de hCG a été réduit de manière significative (environ -50%) par rapport au groupe de souris témoin. Le nombre moyen d'ovocytes libérés chez les groupes de souris qui ont reçu 40 mg/kg d'UPA + 3 mg/kg ou 30 mg/kg de piroxicam est significativement plus faible que le nombre d'ovocytes libérés chez le groupe témoin et chez le groupe 2. De manière remarquable, une dose de 3 mg/kg de piroxicam potentialise l'effet d'inhibition de l'ovulation de l'UPA, alors qu'aucun effet significatif sur l'ovulation n'a été détecté à cette dose de piroxicam (et en l'absence d'UPA) (voir groupe 2 de la série d'expérience 1). Ceci démontre un effet de synergie sur l'inhibition de la rupture folliculaire résultant de l'association de l'UPA et du piroxicam.

La figure 3 montre les coupes histologiques d'ovaires pour des souris de chaque groupe de souris de la série 2. De manière remarquable, les coupes histologiques pour les groupes 3 et 4 révèlent un nombre important de follicules ovariens non-rompus et très peu, voire aucun, corps jaune (*corpus luteum*). Les corps jaunes sont plus nombreux chez les souris ayant reçu de l'UPA en absence de piroxicam. Les coupes histologiques ovariennes du groupe témoin mettent en évidence la présence de nombreux corps jaunes. Ces observations histologiques sont cohérentes avec les résultats relatifs au nombre de follicules libérés et confirment que la combinaison UPA-piroxicam permet d'inhiber ou de retarder l'ovulation et ceci de manière plus efficace que l'administration d'UPA en l'absence d'AINS.

### b) Effet d'inhibition de l'ovulation du meloxicam en combinaison avec l'ulipristal acétate

Un protocole analogue à celui utilisé pour la combinaison piroxicam-ulipristal acétate a été mis en oeuvre pour évaluer l'effet d'inhibition de l'ovulation de la combinaison meloxicam-ulipristal acétate.

Les séries d'expériences suivantes ont été réalisées :
Série 1 :
   - Groupe 1 : placebo
   - Groupe 2 : 3 mg/ kg de piroxicam
   - Groupe 3 : 10 mg/kg de piroxicam
   - Groupe 4 : 30 mg/kg de piroxicam

Série 2 :
- Groupe 1 : placebo
- Groupe 2 : 40 mg/kg d'ulipristal acétate
- Groupe 3 : 40 mg/kg d'ulipristal acétate + 3 mg/kg de piroxicam
- Groupe 4 : 40 mg/kg d'ulipristal acétate + 30 mg/kg de piroxicam

### Résultats :

Les figures 4 et 5 montrent les résultats obtenus pour les séries d'expérience 1 et 2, respectivement.

La figure 4 montre que le meloxicam, lorsqu'il est administré seul n'inhibe que faiblement l'ovulation. Un effet significatif par rapport au groupe témoin n'est observé que pour l'administration d'une dose de 30 mg de meloxicam. La figure 5 montre que l'administration de meloxicam en combinaison avec l'UPA permet de diminuer de manière significative le nombre d'ovocytes libérés par rapport au groupe témoin. L'administration de 30 mg/kg de meloxicam potentialise de manière très significative l'effet d'inhibition de l'ovulation de l'UPA chez le modèle de sur-ovulation étudié. Ceci ressort clairement de la comparaison des résultats obtenus pour le groupe 2 avec ceux obtenus pour le groupe 4.

Les résultats obtenus mettent en évidence que les inhibiteurs de cyclo-oxygénase, tels que les composés appartenant à la famille des oxicams, sont capables de potentialiser l'effet d'inhibition de l'ovulation de l'UPA.

### c) Effet d'inhibition de l'ovulation de l'ibuprofène en combinaison avec l'ulipristal acétate

Un protocole analogue à celui utilisé pour évaluer l'effet de la combinaison piroxicam-ulipristal acétate a été mis en oeuvre pour évaluer la combinaison ibuprofène-ulipristal acétate.

Les séries d'expériences suivantes ont été réalisées :
Série 1 :
   - Groupe 1 : placebo
   - Groupe 2 : 15 mg/kg d'ibuprofène
   - Groupe 3 : 45 mg/kg d'ibuprofène
   - Groupe 4 : 150 mg/kg d'ibuprofène
Série 2 :
   - Groupe 1 : placebo
   - Groupe 2 : 40 mg/kg d'ulipristal acétate
   - Groupe 3 : 40 mg/kg d'ulipristal acétate + 15 mg/kg d'ibuprofène
   - Groupe 4 : 40 mg/kg d'ulipristal acétate + 150 mg/kg d'ibuprofène

### Résultats :

Les figures 6 et 7 montrent les résultats obtenus pour les séries d'expérience 1 et 2, respectivement.

La figure 6 illustre que l'administration d'une dose comprise entre 15 mg et 150 mg d'ibuprofène n'a pas d'effet significatif sur l'ovulation chez le modèle d'hyperstimulation ovarienne étudié.

Comme illustré par la figure 7, on observe une diminution significative du nombre d'ovocytes libérés dans les groupes 2, 3 et 4 par rapport au groupe témoin. L'administration de la combinaison ulipristal acétate-ibuprofène est plus efficace que l'ulipristal acétate, administré seul, pour inhiber l'ovulation.

### Conclusion

La figure 8 résume l'effet de l'UPA et des différentes combinaisons UPA-AINS testées sur l'inhibition de l'ovulation chez le modèle d'hyperstimulation ovarienne étudié. Il apparaît que les combinaisons UPA-AINS sont significativement plus efficaces pour prévenir la rupture folliculaire que l'UPA seul. Par ailleurs, il ressort des résultats présentés ci-avant que les anti-inflammatoires non-stéroidiens testés permettent de potentialiser l'action d'inhibition de la rupture folliculaire de l'ulipristal acétate, l'AINS et l'UPA agissant en synergie.

## Revendications

1. Composition pharmaceutique comprenant
- au moins un modulateur sélectif du récepteur à la progestérone (SPRM) choisi dans le groupe constitué par l'ulipristal acétate, et ses métabolites choisis parmi : 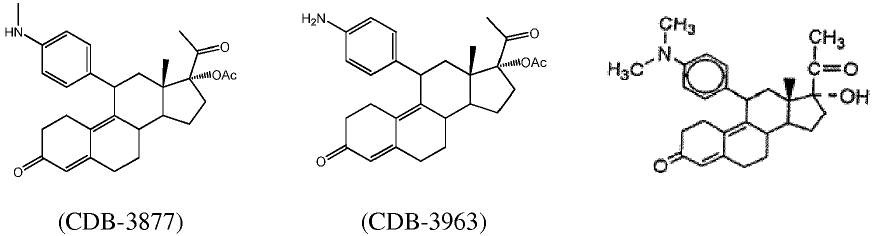 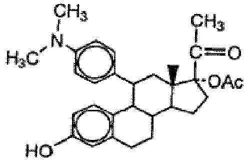 et
- au moins un composé anti-inflammatoire non stéroïdien choisi parmi les inhibiteurs de cyclo-oxygénase de type 2 (COX-2).

2. Composition pharmaceutique selon la revendication 1, dans laquelle le SPRM est choisi dans le groupe constitué par l'ulipristal acétate, CDB-3877 et CDB-3963, de préférence l'ulipristal acétate.

3. Composition pharmaceutique selon l'une des revendications 1 à 2, dans laquelle le composé anti-inflammatoire non stéroïdien est un inhibiteur de cyclo-oxygénase de type 2 choisi parmi les oxicams.

4. Composition selon la revendication 3, dans laquelle l'inhibiteur de cyclo-oxygénase de type 2 est choisi parmi le piroxicam et le meloxicam.

5. Composition pharmaceutique selon la revendication 3, dans laquelle le composé anti-inflammatoire non stéroïdien est un inhibiteur de cyclo-oxygénase de type 2 (COX-2) choisi dans le groupe consistant en l'indométacine, le naproxène, le naproxinod, l'ibuprofène, l'acétominophène, l'étodolac et le célécoxib.

6. Composition pharmaceutique selon l'une des revendications 1 à 5, destinée à une administration par voie orale.

7. Méthode de contraception chez la femme, comprenant l'administration d'une combinaison d'au moins un modulateur sélectif du récepteur à la progestérone (SPRM) choisi dans le groupe constitué par l'ulipristal acétate, et ses métabolites choisis parmi : 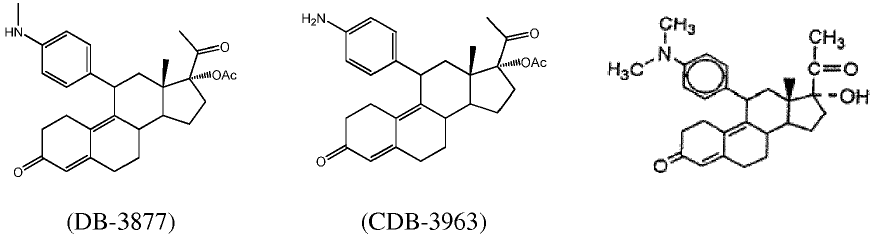 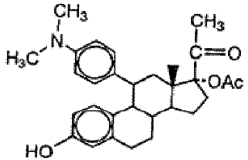 avec au moins un composé anti-inflammatoire non stéroïdien choisi parmi les inhibiteurs de cyclo-oxygénase de type 2 (COX-2).

8. Méthode de contraception selon la revendication 7, dans laquelle le SPRM est l'ulipristal acétate.

9. Méthode de contraception selon la revendication 7 ou 8, dans laquelle le composé anti-inflammatoire non-stéroïdien est un inhibiteur de cyclo-oxygénase de type 2 (COX-2) choisi parmi les oxicams.

10. Méthode de contraception selon la revendication 9, dans laquelle le composé anti-inflammatoire non-stéroïdien est le piroxicam ou le meloxicam.

11. Méthode de contraception selon l'une des revendications 7 ou 8, dans laquelle le composé anti-inflammatoire non stéroïdien est un inhibiteur de cyclo-oxygénase de type 2 (COX-2) choisi dans le groupe constitué par l'indométacine, le naproxène, le naproxinod, l'ibuprofène, l'acétominophène, l'étodolac et le célécoxib.

12. Méthode de contraception selon l'une des revendications 7 à 11, le SPRM, et le composé anti-inflammatoire non stéroïdien étant administrés de manière séparée, simultanée ou séquentielle.

13. Méthode de contraception selon l'une quelconque des revendications 7 à 12, dans laquelle le SPRM, et le composé anti-inflammatoire non stéroïdien sont administrés par voie orale.

14. Méthode de contraception selon l'une des revendications 7 à 13, ladite méthode étant une méthode de contraception d'urgence ou une méthode de contraception régulière

## Patentansprüche

1. Pharmazeutische Zusammensetzung, umfassend:
- wenigstens einen selektiven Progesteronrezeptor-Modulator (SPRM), ausgewählt aus der Gruppe, bestehend aus Ulipristalacetat und seinen Metaboliten, ausgewählt aus: 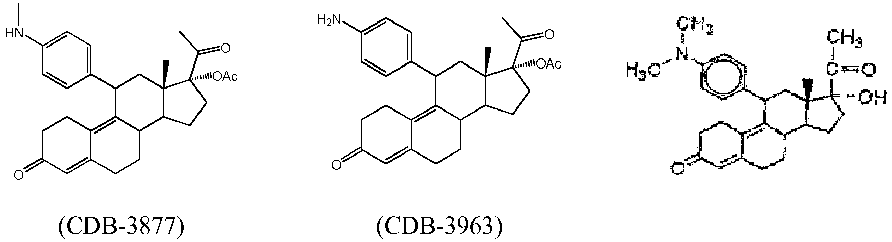 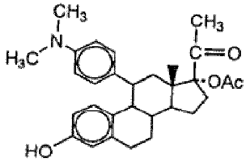 und
- wenigstens eine nichtsteroidale entzündungshemmende Verbindung, ausgewählt aus den Hemmern von Cyclooxygenase-2 (COX-2).

2. Pharmazeutische Zusammensetzung gemäß Anspruch 1, wobei der SPRM aus der Gruppe ausgewählt ist, bestehend aus Ulipristalacetat, CDB-3877 und CDB-3963, vorzugsweise Ulipristalacetat.

3. Pharmazeutische Zusammensetzung gemäß einem der Ansprüche 1 bis 2, wobei die nichtsteroidale entzündungshemmende Verbindung ein Hemmer von Cyclooxygenase-2 ist, ausgewählt aus den Oxicamen.

4. Zusammensetzung gemäß Anspruch 3, wobei der Hemmer von Cyclooxygenase-2 aus Piroxicam und Meloxicam ausgewählt ist.

5. Pharmazeutische Zusammensetzung gemäß Anspruch 3, wobei die nichtsteroidale entzündungshemmende Verbindung ein Hemmer von Cyclooxygenase-2 (COX-2) ist, ausgewählt aus der Gruppe, bestehend aus Indometacin, Naproxen, Naproxinod, Ibuprofen, Acetominophen, Etodolac und Celecoxib.

6. Pharmazeutische Zusammensetzung gemäß einem der Ansprüche 1 bis 5, für eine orale Verabreichung.

7. Verfahren zur Empfängnisverhütung bei der Frau, umfassend die Verabreichung einer Kombination aus wenigstens einem selektiven Progesteronrezeptor-Modulator (SPRM), ausgewählt aus der Gruppe, bestehend aus Ulipristalacetat und seinen Metaboliten, ausgewählt aus: 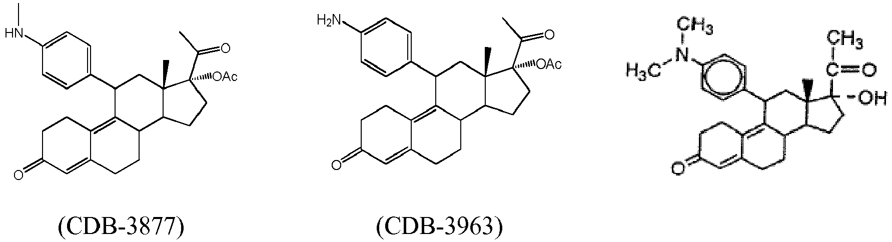 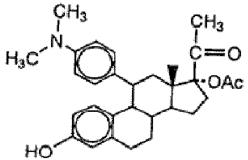 mit wenigstens einer nichtsteroidalen entzündungshemmenden Verbindung, ausgewählt aus den Hemmern von Cyclooxygenase-2 (COX-2).

8. Verfahren zur Empfängnisverhütung gemäß Anspruch 7, wobei der SPRM Ulipristalacetat ist.

9. Verfahren zur Empfängnisverhütung gemäß Anspruch 7 oder 8, wobei die nichtsteroidale entzündungshemmende Verbindung ein Hemmer von Cyclooxygenase-2 (COX-2) ist, ausgewählt aus den Oxicamen.

10. Verfahren zur Empfängnisverhütung gemäß Anspruch 9, wobei die nichtsteroidale entzündungshemmende Verbindung Piroxicam oder Meloxicam ist.

11. Verfahren zur Empfängnisverhütung gemäß einem der Ansprüche 7 oder 8, wobei die nichtsteroidale entzündungshemmende Verbindung ein Hemmer von Cyclooxygenase-2 (COX-2) ist, ausgewählt aus der Gruppe, bestehend aus Indometacin, Naproxen, Naproxinod, Ibuprofen, Acetominophen, Etodolac und Celecoxib.

12. Verfahren zur Empfängnisverhütung gemäß einem der Ansprüche 7 bis 11, wobei der SPRM und die nichtsteroidale entzündungshemmende Verbindung getrennt, gleichzeitig oder nacheinander verabreicht werden.

13. Verfahren zur Empfängnisverhütung gemäß einem der Ansprüche 7 bis 12, wobei der SPRM und die nichtsteroidale entzündungshemmende Verbindung oral verabreicht werden.

14. Verfahren zur Empfängnisverhütung gemäß einem der Ansprüche 7 bis 13, wobei besagtes Verfahren ein Verfahren zur Empfängnisverhütung im Notfall oder ein Verfahren zur regelmäßigen Empfängnisverhütung ist.

## Claims

1. A pharmaceutical composition comprising
- at least one selective progesterone receptor modulator (SPRM) selected from the group consisting of ulipristral acetate, and its metabolites selected from: 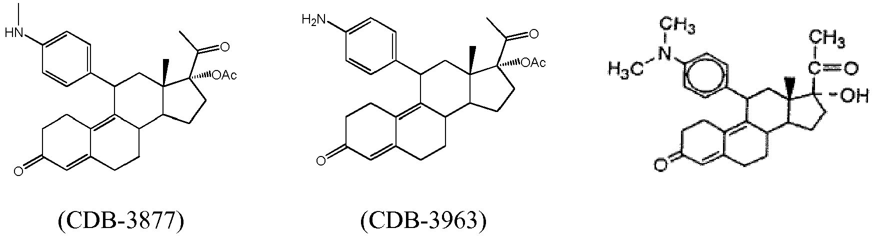 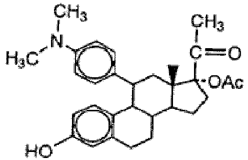 and
- at least one non-steroidal anti-inflammatory compound selected from the cyclooxygenase type 2 (COX-2) inhibitors.

2. The pharmaceutical composition according to Claim 1, wherein the SPRM is selected from the group consisting of ulipristral acetate, CDB-3877 and CDB-3963, preferably ulipristral acetate.

3. The pharmaceutical composition according to any of claims 1 to 2, wherein the non-steroidal anti-inflammatory compound is a cyclooxygenase type 2 inhibitor selected from oxicams.

4. The pharmaceutical composition according to Claim 3, wherein the cyclooxygenase type 2 inhibitor is selected from piroxicam and meloxicam.

5. The pharmaceutical composition according to Claim 3, wherein the non-steroidal anti-inflammatory compound is a cyclooxygenase type 2 (COX-2) inhibitor selected from the group consisting of indomethacin, naproxen, naproxcinod, ibuprofen, acetaminophen, etodolac and celecoxib.

6. The pharmaceutical composition according to any of claims 1 to 5, for oral administration.

7. A method of contraception in a woman, comprising the administration of a combination of at least one selective progesterone receptor modulator (SPRM) selected from the group consisting of ulipristral acetate, and its metabolites selected from: 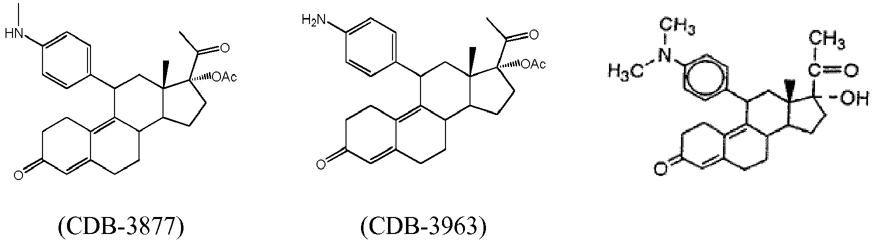 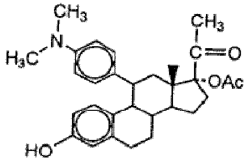 with at least one non-steroidal anti-inflammatory compound selected from the cyclooxygenase type 2 (COX-2) inhibitors.

8. The method of contraception according to Claim 7, wherein the SPRM is ulipristral acetate.

9. The method of contraception according to Claim 7 or 8, wherein the non-steroidal anti-inflammatory compound is a cyclooxygenase type 2 (COX-2) inhibitor selected from oxicams.

10. A method of contraception according to Claim 9, wherein the non-steroidal anti-inflammatory compound is piroxicam or meloxicam.

11. A method of contraception according to any of claims 7 or 8, wherein the non-steroidal anti-inflammatory compound is a cyclooxygenase type 2 (COX-2) inhibitor selected from the group consisting of indomethacin, naproxen, naproxcinod, ibuprofen, acetaminophen, etodolac and celecoxib.

12. A method of contraception according to any of claims 7 to 11, the SPRM, and the non-steroidal anti-inflammatory compound being administrated separately, simultaneously or sequentially.

13. A method of contraception according to any of claims 7 to 12, wherein the SPRM, and the non-steroidal anti-inflammatory compound are orally administrated.

14. A method of contraception according to any of claims 7 to 13, where said method is an emergency contraception method or a regular contraception method.
